# EUROPEAN PATENT SPECIFICATION

(11) **EP 1 787 658 B1**
(45) Date of publication and mention of the grant of the patent: **14.03.2012**
(21) Application number: 05077559.2
(22) Date of filing: 10.11.2005
(51) Int. Cl.: A61K 9/00, A61K 38/31, A61K 47/10, A61K 47/14, A61K 47/24

(54) **Sustained release formulations of somatostatin analogue inhibitors of growth hormone**
Formulierungen mit verzögerter Freisetzung, die Somatostatin-analoge Wachstumshormoninhibitoren enthalten
Formulations à libération prolongée contenant des inhibiteurs de l'hormone de croissance analogues de la somatostatine

(43) Date of publication of application: 23.05.2007
(73) Proprietor: CHEMI S.p.A., 20092 Cinisello Balsamo (Milano) (IT)
(72) Inventor: Mascagni, Paolo, 20058 Villasanta (MI) (IT)
(74) Representative: Fiammenghi-Domenighetti, Delfina

(56) References cited:
- EP-A- 0 988 861
- WO-A-01/89479
- WO-A-94/08610
- WO-A-99/21533
- US-A1- 2003 049 320

## Description

The present invention relates to physically stable, transparent, biologically compatible and easy to prepare lipid solutions of somatostatin analogue inhibitors of growth hormone release.

For the purposes of the present invention, the expression "somatostatin analogue inhibitor of growth hormone release" indicates any biologically active oligopeptide inhibiting the growth hormone release from mammalian pituitary gland. These biologically active oligopeptides are well known in the art, together with the mechanism of action thereof, which is disclosed for instance by Weckbecker et al. Nature Reviews, Vol. 2, pages 999-1016, December 2003 and Murray et al. The Journal of Clinical Investigation, Vol. 114, n. 3, pages 349-356, August 2004. Apart from somatostatin itself, these biologically active oligopeptides include but are not limited to octreotide, lanreotide, vapreotide, pasireofide, PTR-3173, BIM-23268, together with the pharmaceutically acceptable salts thereof, preferably the acetate. For sake of completeness, the formulae of some of these compounds are reported here-below.

Lanreotide acetate

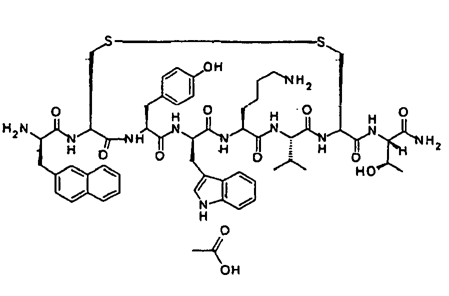

Octreotide acetate

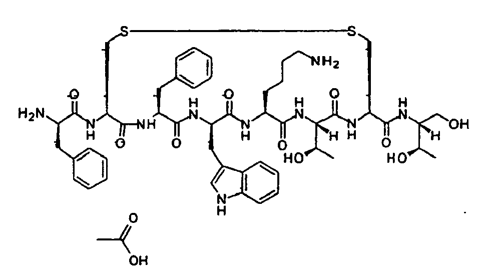

Vapreotide acetate

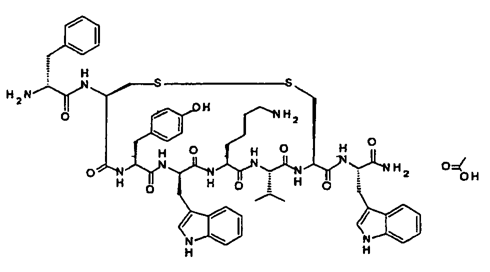

SOM-230 - Pasireotide

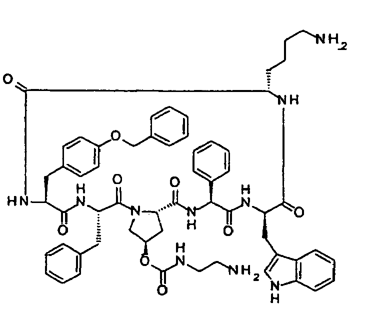

PTR-3173

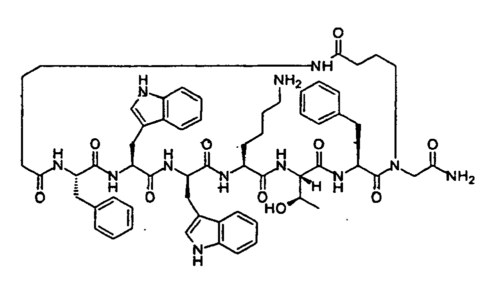

BIM-23268

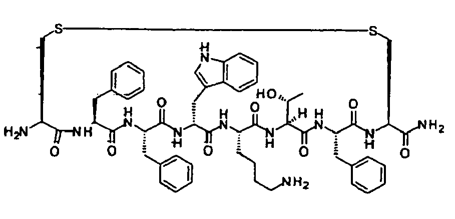

The present invention further relates to the use of said solutions as pharmaceutical composition for the parenteral administration of these specific biologically active oligopeptides, with the aim of obtaining a sustained release of said active ingredient after subcutaneous and/or intramuscular administration. More particularly, the present invention relates to a specific biologically active formulation, obtained by the addition of the oligopeptide to a mixture composed of one or more C₁-C₈-alcohol, in particular ethanol, a phospholipid, in particular lecithin, and one or more fatty acid esters, in particular isopropyl myristate.

### Background of the invention

Parenteral drugs, especially peptides or proteins, which have a short half-life in vivo, require sustained release formulation in order to avoid the use of repeated daily injections or continuous infusion administration. Micro-encapsulation in biodegradable polymers has been disclosed as a suitable mean for drug release control in a number of patent documents [see, inter alia, US6113943 and references cited fherein]. Major disadvantages of polymer formulations are: i) a rather complex manufacturing process, ii) the use of organic, chlorinated, solvents and iii) injection of a solid suspension, iv) preparation of oral suspension.

Microemulsion type formulations have also been proposed as drug delivery system and sustained release preparation. Although microemulsions have been mainly used for oral administration (as in WO 94/08610), their use for the preparation of injectable, sustained release, formulation of hydrophilic drugs has also been disclosed [WO 01/89479; M.R. Gasco et al., Int.J.Pharm., 62, 119 (1990); G.C. Ritthidej et al. "Controlled Release Society 29^{th} annual meeting" poster #696 (2002)]. Microemulsions described in the above cited reference are said to allow a sustained release of the active ingredient that lasts 3 or 4 weeks.

Lecithin is cited and used in several kinds of solutions or injectable systems (Int J Pharm, 116 (2),253-261, 1995 inter alia, WO 99/29301, Int J Pharm, 143, 67-73,1996 Trotta et al.); ethanol and isopropyl mirystate are also cited as excipients used for some microemulsions; these systems contain a certain percentage of water.

Formulations for the sustained release of somatostatin analogue inhibitors of growth hormone release are disclosed for instance in US5538739, WO 95/06077, WO 93/20126, WO 2002/009739, WO 2001/037784, WO 97/47317, WO 95/00126, EP816413, US5753618, US5656721, GB2193891, US5393738, AU574081, US4451452, WO 2004/045633, WO 2004/052340, WO 2003/089008, US4801739, US5863549.

### Detailed description of the invention

The object of the present invention is to provide a physically stable, transparent, biologically compatible and easy to prepare lipid solutions of somatostatin analogue inhibitors of growth hormone release.

It has now been surprisingly found that, although these oligopeptides are not soluble in isopropyl myristate, ethanol, lecithin or binary mixtures thereof, they are however soluble in a mixture containing a C₁-C₈-alcohol, a phospholipid and a C₁-C₄ alkyl fatty acid ester, thus leading to a clear, physically stable, transparent solution.

The object of the present invention is therefore represented by a pharmaceutical formulation in the form of a lipid solution essentially consisting of at least one somatostatin analogue inhibitor of growth hormone release, a C₁-C₈ alcohol, a phospholipid and a C₁-C₄ alkyl fatty acid ester.

According to a preferred embodiment of the invention, the C₁-C₈-alcohol is a C₁-C₄-alcohol, preferably ethanol; the phospholipid is lecithin, preferably soybean lecithin; and the C₁-C₄ alkyl fatty acid ester is selected from isopropyl myristate, ethyl oleate, ethyl myristate, ethyl palmitate, ethyl stearate, ethyl oleate, miglycol, sesame oil and oleic acid, sesame oil and gliceryl monostearate, isopropyl palmitate, ter-butyl myristate; according to the best embodiment of the invention it is a C₁-C₄ alkyl myristate, preferably isopropyl myristate. Such a preferred formulation is a biologically active and biologically compatible solution, which is clear, has a very low viscosity and a density ranging from 0.80 to 1.10 gr/cm³.

The somatostatin analogue inhibitor of growth hormone release is normally present in amounts ranging from 0.1 to 10% w/w, more in particular from 1 to 4% w/w, preferably from 1 to 3% w/w.

The C₁-C₄ alkyl fatty acid ester, in particular isopropyl myristate, is present in amounts ranging from 10 to 80% w/w, more in particular from 20 to 70% w/w, preferably from 40 to 65% w/w.

The C₁-C₈-alcohol, in particular ethanol, is present in amounts ranging from 1 to 20% w/w, more in particular from 5 to 10% w/w, preferably about 7% w/w.

The phospholipid, more specifically soybean lecithin, is present in amounts ranging from 5 to 50% w/w, more in particular from 10 to 40% w/w, preferably from 20 to 30 %w/w.

The somatostatin analogue inhibitor of growth hormone release, the C₁-C₄ alkyl fatty acid ester, the C₁-C₄-alcohol and the phospholipid are normally present in amounts summing up to 100% (i.e. the composition consists of these four components).

The process for the preparation of said formulation is the following:

Manufacturing is generally performed on weight basis; the process can be performed by simple admixture of the components at room temperature (i.e. at 10-40° C, preferably at 15-30° C) with no additional heating required and without using additional solvents; it can be performed by weighing the specific active ingredient employed in this invention together with all the other ingredients of such formulation. The mixture can then be stirred magnetically or mechanically; the process can last from 30min to 24 hours, depending on the batch size.

The described formulation can be prepared ranging from milligrams to kilos, from lab to production scale.

Another objective of the present invention relates to the possibility of preparing the solution just prior to injection; it was found that the specific biologically active peptide can be lyophilized without adding any bulking agent in the final primary packaging. The lyophilized powder is then turned into a transparent, clear solution by adding to the lyophilized powder the precise amount or volume of the inactive ingredients mixture cited above, stored in a different vial or in a pre-filled syringe or other suitable container. The solution is formed immediately or in a few minutes.

Another objective of the present invention is said formulation for use in a therapeutical treatment comprising the sustained release (upon subcutaneous or intramuscular injection to a mammalian of a dosage of the active principle of from 10 to 20 mg/kg of the mammalian itself) of the active oligopeptide, which lasts for at least 20 days, preferably 35 days, more preferably 40. More in particular, and as it will be apparent from the examples, this solution is able to control the delivery of the active ingredient in the blood of a mouse for at least 49 days; more in particular, plasma profiles of the monitored active ingredients in rats indicate the presence of pharmacologically active levels of the oligopeptide for at least 49 days; therefore, the solution according to the present invention, either ready to use or reconstituted prior to injection, can be administered once every month, in order to safely control the release of the active oligopeptide for 30 days.

According to one of the preferred embodiments, a single-dosage formulation according to the invention would normally contain from 10 to 60 mg of the somatostatin analogue inhibitor of growth hormone release, preferably from 20 to 40 mg

### Example 1: lipid solution composition

The table below (Table 1) describes an example of the lipid solution, primary objective of the present invention.

**Table 1**

| Composition | w/w percentage |
|---|---|
| Octreotide acetate | 3.37 |
| Soybean lecithin | 27.00 |
| Isopropyl myristate | 62.63 |
| Ethanol | 7.00 |

The biologically active and compatible solution is transparent, clear, with a very low viscosity and a density of 0.89 gr/cm³.

Two groups of animals were treated with a single administration each s.c. and i.m, of 100µl of the formulation (3.37 mg of oligopeptide equal to 15mg/Kg). Blood samples from 5 animals per time point were collected at different time points up to 49 days after treatment. The plasma levels of somatostine analogue were determined by a LC/MS-MS method.

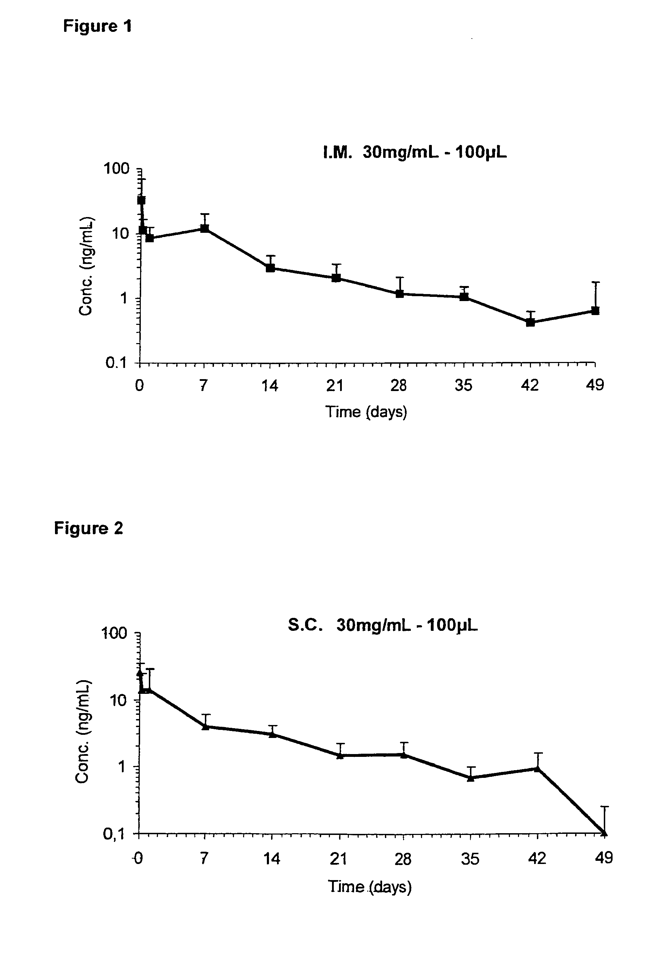

Mean plasma, levels and standard deviations for each time point are shown in the table below (Table 2) and in Figures 1 and 2.

**Table 2**

| | **Time** | | | | | | | | | |
|---|---|---|---|---|---|---|---|---|---|---|
| | 1h | 6h | 24h | 7gg | 14gg | 21gg | 28gg | 35gg | 42gg | 49gg |
| **Formulation 30mg/ml Intramuscolar (TF04/275) 15mg/kg** | | | | | | | | | | |
| Media | 33.10 | 11.33 | 8.55 | 12.02 | 2.97 | 2.06 | 1.17 | 1.04 | 0.42 | 0.63 |
| D.S. | 35.95 | 5.38 | 4.06 | 8.30 | 1.63 | 1.32 | 0.92 | 0.45 | 0.20 | 1.10 |

| **Formulation 30mg/ml Sub-cutaneous (TF04/251) 15mg/kg** | | | | | | | | | | |
|---|---|---|---|---|---|---|---|---|---|---|
| Media | 25.85 | 13.93 | 13.85 | 4.01 | 3.03 | 1.50 | 1.56 | 0.70 | 0.94 | 0.10 |
| D.S. | 9.17 | 10.25 | 14.48 | 1.95 | 1.11 | 0.74 | 0.80 | 0.33 | 0.67 | 0.15 |

The presence of detectable levels of the oligopeptide up to 49 days after both s.c. and i.m injection.

The pharmacodynamic profile of the formulation has also been evaluated in rats. The efficacy of the preparation has been evaluated as inhibitory effect of the barbituric-induced growth hormone (GH) production over a period of 4 weeks.

In the present study the animals (male, 200 gr Sprague-Dawley) were divided in two groups. The first group was treated with Placebo (30 µl s.c.), whereas the second with 4.5 mg/Kg of active principle (30 µl s.c.). On days 1, 3, 7, 14, 21 the animals were treated with barbituric (60 mg/Kg i.p.) and 30 minutes later blood collected. The GH in the serum was quantified by commercial kit specific for the rat. On day 28 the animals were submitted to a second administration of the formulation or Placebo and barbituric-induced GH production evaluated on day 29, 31 and 35.

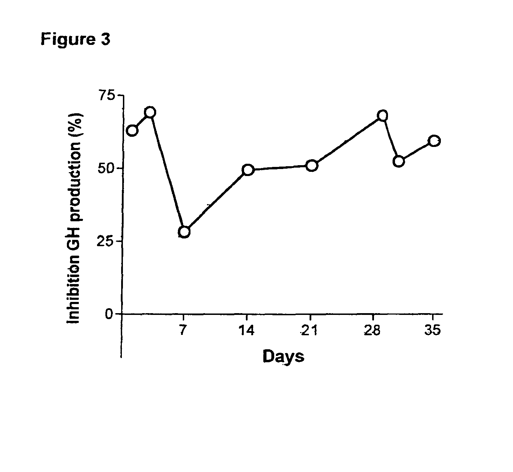

The efficacy of the treatment is expressed as percent inhibition of GH production in respect to the control group and the results obtained are summarized in the Figure 3. The results obtained indicate that the formulation potently inhibits the production of GH by about 70 percent just after 24 hours after treatment and, then, maintaining its efficacy throughout the entire period of the study.

### Example 2: lipid solution composition

The table below (Table 3) describes a further example of the lipid solution, primary objective of the present invention.

**Table 3**

| Composition | w/w percentage |
|---|---|
| Octreotide acetate | 2.00 |
| Soybean lecithin | 27.00 |
| Isopropyl myristate | 64.00 |
| Ethanol | 7.00 |

### Example 3: lipid solution composition

The table below (Table 4) describes a further example of the lipid solution, primary objective of the present invention.

**Table 4**

| Composition | w/w percentage |
|---|---|
| Octreotide acetate | 5.00 |
| Soybean lecithin | 27.00 |
| Isopropyl myristate | 61.00 |
| Ethanol | 7.00 |

### Example 4: laboratory scale manufacturing process of the solution

Soybean lecithin, ethanol, isopropyl myristate are weighed in a suitable glass beaker; the prescribed amount of octreotide acetate is then added to the mixture under stirring; filtered nitrogen is bubbled rapidly, then the container is closed and left under magnetic stirring at room temperature for 12-14 hours, or anyway until the solution is completely clear.

The solution is then left at rest for 2 hours without any stirring.

The solution is filtered using a glass syringe equipped with polyvinylidene fluoride pre-sterilized disposable glass filter (pore size 0,22µm). Filtration is performed under laminar flow to prevent microbial contamination. The solution is inspected for transparency and absence of particulate before and after filtration, as "in process control".

### Example 5: reconstitution of the lyophilized oligopeptide

The peptide is stored as a lyophilized powder in a vial; powder amount can range from 10 to 30mg/vial.

The solution consisting of soybean lecithin, ethanol, isopropyl myristate is stored in vials or in pre-filled syringe, in a volume ranging from 0.5 to 5 ml, more preferably 1.2 ml.

The inactive solution is then transferred into the vial containing the lyophilized peptide powder; the vial is then gently, manually shacked. A clear, transparent and biologically active solution is formed.

## Claims

1. A pharmaceutical formulation in the form of a lipid solution consisting essentially of at least one somatostatin analogue inhibitor of growth hormone release, a C₁-C₈ alcohol, a phospholipid and a C₁-C₄ alkyl fatty acid ester.

2. The pharmaceutical formulation according to claim 1, wherein the somatostatin analogue inhibitor of growth hormone release is present in amounts of from 0.1 to 10% by weight, the C₁-C₄ alkyl fatty acid ester is present in amounts of from 10 to 80% by weight, the C₁-C₈ alcohol is present in amounts of from 1 to 20% by weight and the phospholipid is present in amounts of from 5 to 50% by weight.

3. The pharmaceutical formulation according to any of the above claims, wherein the somatostatin analogue inhibitor of growth hormone release is present in amounts of from 1 to 4% by weight, preferably from 1 to 3%.

4. The pharmaceutical formulation according to any of the above claims, wherein the C₁-C₄ alkyl fatty acid ester is present in amounts of from 20 to 70% by weight, preferably from 40 to 65%.

5. The pharmaceutical formulation according to any of the above claims, wherein the C₁-C₈ alcohol is present in amounts of from 5 to 10% by weight, preferably about 7%.

6. The pharmaceutical formulation according to any of the above claims, wherein the phospholipid is present in amounts of from 10 to 40% by weight, preferably from 20 to 30%.

7. The pharmaceutical formulation according to claims 2 to 6, wherein the somatostatin analogue inhibitor of growth hormone release, the C₁-C₄ alkyl fatty acid ester, the C₁-C₈ alcohol and the phospholipid sum up to 100%.

8. The pharmaceutical formulation according to any of the above claims, wherein the somatostatin analogue inhibitor of growth hormone release is selected from somatostatin, octreotide, lanreotide, vaprotide, pasireotide, PTR-3173, BIM-23268 and the pharmaceutically acceptable salts thereof, preferably the acetate.

9. The pharmaceutical formulation according to any of the above claims, wherein the C₁-C₈-alcohol is a C₁-C₄-alcohol.

10. The pharmaceutical formulation according to any of the above claims, wherein the C₁-C₈-alcoholis ethanol.

11. The pharmaceutical formulation according to any of the above claims, wherein the phospholipid is lecithin, preferably soybean lecithin.

12. The pharmaceutical formulation according to any of the above claims, wherein the phospholipid is obtained via a synthetic or semi-synthetic chemical process and the fatty acids in positions 1 and 2 of glycerol are saturated or unsaturated.

13. The pharmaceutical formulation according to any of the above claims, wherein the C₁-C₄ alkyl fatty acid ester is selected from isopropyl myristate, ethyl myristate, ethyl palmitate, ethyl stearate, ethyl oleate, miglycol, sesame oil and oleic acid, sesame oil and gliceryl monostearate, isopropyl palmitate, ter-butil myristate.

14. The pharmaceutical formulation according to any of the above claims, wherein the C₁-C₄ alkyl fatty acid ester is a C₁-C₄ alkyl myristate.

15. The pharmaceutical formulation according to any of the above claims, wherein the C₁-C₄ alkyl fatty acid ester is isopropyl myristate.

16. The pharmaceutical formulation according to any of the above claims **characterized by** being a transparent solution.

17. A kit for the extemporaneous preparation of a pharmaceutical formulation according to any of the above claims, which comprises a first container containing a somatostatin analogue inhibitor of growth hormone release and a second container containing a lipid solution consisting essentially of C₁-C₈ alcohol, the phospholipid and the C₁₋C₄ alkyl fatty acid ester.

18. A kit according to claim 17 which contains from 10 to 60 mg of the somatostatin analogue inhibitor of growth hormone release, preferably from 20 to 40 mg.

19. A kit according to claim 17 or 18 wherein the somatostatin analogue inhibitor of growth hormone release is in lyophilized form.

20. A pharmaceutical formulation according to anyone of claims 1-16 for use in a therapeutical treatment comprising the sustained release of a somatostatin analogue inhibitor of growth hormone by a single subcutaneous or intramuscular administration of said composition once every at least 20 days, preferably 35 days.

21. A pharmaceutical formulation according to claim 20, wherein the single subcutaneous or intramuscular administration of said composition is made once every at least 40 days, preferably 49.

22. A pharmaceutical formulation according to claim 20 or 21, wherein from 10 to 100 mg/ml of the somatostatin analogue inhibitor of growth hormone release are administered.

23. A pharmaceutical formulation according to claim 22, wherein from 10 to 60 mg/ml of the somatostatin analogue inhibitor of growth hormone release are administered.

24. A pharmaceutical formulation according to claim 23, wherein a single dosage of 20 to 40 mg of the somatostatin analogue inhibitor of growth hormone release is administered.

## Patentansprüche

1. Pharmazeutische Formulierung in Form einer Lipid-Lösung, die im Wesentlichen aus einem Analogon des Somatostatin als Hemmer der Wachstumshormonfreisetzung, einem C₁-C₈ - Alkohol, einem Phospholipid und einem C₁-C₄ -Fettsäurealkylester besteht.

2. Pharmazeutische Formulierung nach Anspruch 1, in der das Analogon des Somatostatin als Hemmer der Wachstumshormonfreisetzung in Mengen von 0,1 bis 10 Gew-% vorliegt, der Fettsäurealkylester in Mengen von 10 bis 80 Gew-% vorliegt, der C₁-C₈ - Alkohol in Mengen von 1 bis 20 Gew-% vorliegt und das Phospholipid in Mengen von 5 bis 50 Gew-% vorliegt.

3. Pharmazeutische Formulierung nach den vorstehenden Ansprüchen, in der das Analogon des Somatostatin als Hemmer der Wachstumshormonfreisetzung in Mengen von 1 bis 4 Gew-%, vorzugsweise von 1 bis 3 Gew-%, vorliegt.

4. Pharmazeutische Formulierung nach den vorstehenden Ansprüchen, in der der Fettsäurealkylester in Mengen von 20 bis 70 Gew-%, vorzugsweise von 40 bis 65 Gew-%, vorliegt.

5. Pharmazeutische Formulierung nach den vorstehenden Ansprüchen, in der der C₁-C₈ - Alkohol in Mengen von 5 bis 10 Gew-%, vorzugsweise von ca. 7 Gew-%, vorliegt.

6. Pharmazeutische Formulierung nach den vorstehenden Ansprüchen, in der das Phospholipid in Mengen von 10 bis 40 Gew-%, vorzugsweise von 20 bis 30 Gew-%, vorliegt.

7. Pharmazeutische Formulierung nach den Ansprüchen von 2 bis 6, in der das Analogon des Somatostatin als Hemmer der Wachstumshormonfieisetzung, der C₁-C₄ - Fettsäurealkylester, der C₁-C₈- Alkohol und das Phospholipid zusammen das 100% betragen.

8. Pharmazeutische Formulierung nach den vorstehenden Ansprüchen, in der das Analogon des Somatostatin als Hemmer der Wachstumshormonfreisetzung aus der Gruppe bestehend aus Somatostatin, Octreotid, Lanreotid, Vaprotid, Pasireotid, PTR-3173, B1M 23268 und deren pharmazeutisch verwendbare Salze, vorzugsweise deren Azetat, ausgewählt ist.

9. Pharmazeutische Formulierung nach den vorstehenden Ansprüchen, in der der C₁-C₈ - Alkohol ein C₁-C₄-Alkohol ist.

10. Pharmazeutische Formulierung nach den vorstehenden Ansprüchen, in der der C₁-C₈ - Alkohol Ethanol ist.

11. Pharmazeutische Formulierung nach den vorstehenden Ansprüchen, in der das Phospholipid Lezithin, vorzugsweise Sojalezithin, ist.

12. Pharmazeutische Formulierung nach den vorstehenden Ansprüchen, in der das Phospholipid mit einem synthetischen oder semi-synthetischen chemischen Verfahren hergestellt wird und die Fettsäuren an den Stellen 1 und 2 des Glycerols gesättigt oder ungesättigt sind.

13. Pharmazeutische Formulierung nach den vorstehenden Ansprüchen, in der der C₁-C₄-Fettsäurealkylester aus der Gruppe bestehend aus Isopropylmyristat, Ethylmyristat, Ethylpalmitat, Ethylstearat, Ethyloleat, Miglycol, Sesamöl und Oleinsäure, Sesamöl und Glyzerinmonostearat, Isopropylpalmitat, Tert-Butylmyristat, ausgewählt ist.

14. Pharmazeutische Formulierung nach den vorstehenden Ansprüchen, in der der C₁-C₄ - Fettsäurealkylester C₁-C₄ -Alkylmyristat ist.

15. Pharmazeutische Formulierung nach den vorstehenden Ansprüchen, in der der C₁-C₄ - Fettsäurealkylester Isopropylmyristat ist.

16. Pharmazeutische Formulierung nach den vorstehenden Ansprüchen, **dadurch gekennzeichnet dass** diese eine durchsichtige Lösung ist.

17. Kit für die unmittelbare Vorbereitung einer pharmazeutischen Formulierung nach den voranstehenden Ansprüchen, bestehend aus einem ersten Behälter, der das Analogon des Somatostatin als Hemmer der Wachstumshormonfreisetzung enthält, und aus einem zweiten Behälter, der eine Lipid-Lösung enthält, die im Wesentlichen aus einem C₁-C₈ A -Alkohol, einem Phospholipid und einem C₁-C₄-Fettsäurealkylester besteht.

18. Kit nach Anspruch 17, der von 10 bis 60 mg, vorzugsweise von 20 bis 40 mg, des Analogons des Somatostatin als Hemmer der Wachstumshormonfreisetzung enthält.

19. Kit nach Anspruch 17 oder 18, in der das Analogon des Somatostatin als Hemmer der Wachstumshormonfreisetzung in lyophilisierter Form ist.

20. Pharmazeutische Formulierung nach einem der Ansprüche 1-16, für die Verwendung in einer therapeutischen Behandlung, die die kontinuierliche Abgabe von dem Analogon des Somatostatin als Hemmer des Wachstumshormons, mittels einer einzigen subkutanen oder intramuskulären Verabreichung der genannten Zusammensetzung mindestens einmal jede 20 Tage, vorzugsweise 35 Tage, umfasst.

21. Pharmazeutische Formulierung nach Anspruch 20, in der die subkutane oder intramuskuläre Verabreichung der genannten Zusammensetzung einmal jede mindestens 40 Tage, vorzugsweise 49 Tage, gegeben wird.

22. Pharmazeutische Formulierung nach Anspruch 20 oder 21, in der von 10 bis 100 mg/ml des Analogons des Somatostatin als Hemmer der Wachstumshormonfreisetzung verabreicht werden.

23. Pharmazeutische Formulierung nach Anspruch 22, in der von 10 bis 60 mg/ml des Analogons des Somatostatin als Hemmer der Wachstumshormonfreisetzung verabreicht werden.

24. Pharmazeutische Formulierung nach Anspruch 23, in der eine einzige Dosierung von 20 bis 40 mg/ml des Analogon des Somatostatin als Hemmer der Wachstumshormonfreisetzung verabreicht wird.

## Revendications

1. Formulation pharmaceutique sous forme de solution lipidique, qui consiste essentiellement en un analogue de la somatostatine comme inhibiteur de la libération de l'hormone de croissance, un alcool en C₁-C₈, un phospholipide et un ester alkylique d'acide gras en C₁ -C₄.

2. Formulation pharmaceutique selon la revendication 1, dans laquelle l'analogue de la somatostatine comme inhibiteur de la libération de l'hormone de croissance est présent en quantités de 0,1 à 10% en poids, l'ester alkylique d'acide gras en C₁-C₄ est présent en quantités de 10 à 80% en poids, l'alcool en C₁-C₈ est présent en quantités de 1 à 20% en poids et le phospholipide est présent en quantités de 5 à 50% en poids.

3. Formulation pharmaceutique selon quelconque des revendications précédentes, dans laquelle l'analogue de la somatostatine comme inhibiteur de la libération de l'hormone de croissance est présent en quantités de 1 à 4% en poids, de préférence de 1 à 3%.

4. Formulation pharmaceutique selon quelconque des revendications précédentes, dans laquelle l'ester alkylique d'acide gras en C₁-C₄ est présent en quantités de 20 à 70% en poids, de préférence de 40 à 65%.

5. Formulation pharmaceutique selon quelconque des revendications précédentes, dans laquelle l'alcool en C₁-C₈ est présent en quantités de 5 à 10% en poids, de préférence environ 7%.

6. Formulation pharmaceutique selon quelconque des revendications précédentes, dans laquelle le phospholipide est présent en quantités de 10 à 40% en poids, de préférence de 20 à 30%.

7. Formulation pharmaceutique selon les revendications de 2 à 6, dans laquelle l'analogue de la somatostatine comme inhibiteur de la libération de l'hormone de croissance, l'ester alkylique d'acide gras en C₁-C₄, l'alcool en C₁-C₈ et le phospholipide représentent ensemble le 100%.

8. Formulation pharmaceutique selon quelconque des revendications précédentes, dans laquelle l'analogue de la somatostatine comme inhibiteur de la libération de l'hormone de croissance est sélectionné parmi somatostatine, octreonide, lanreotide, vaprotide, pasireotide, PTR-3173, BIM-23268 et leurs sels pharmaceutiquement acceptables, de préférence l'acétate.

9. Formulation pharmaceutique selon quelconque des revendications précédentes, dans laquelle l'alcool en C₁-C₈ est un alcool en C₁-C₄.

10. Formulation pharmaceutique selon quelconque des revendications précédentes, dans laquelle l'alcool en C₁-C₈ est éthanol.

11. Formulation pharmaceutique selon quelconque des revendications précédentes, dans laquelle le phospholipide est lécithine, de préférence lécithine de soja.

12. Formulation pharmaceutique selon quelconque des revendications précédentes, dans laquelle le phospholipide est obtenu par un procédé chimique synthétique ou partiellement synthétique et les acides gras sont saturés ou insaturés en positions 1 et 2 du glycérol.

13. Formulation pharmaceutique selon quelconque des revendications précédentes, dans laquelle l'ester alkylique d'acide gras en C₁ -C₄ est sélectionné parmi isopropyle myristate, éthyle myristate, éthyle palmitate, éthyle stéarate, éthyle oléate, Miglycol, huile de sésame et acide oléique, huile de sésame et monostéarate de glycéryle, isopropyle palmitate, tert-butyl myristate.

14. Formulation pharmaceutique selon quelconque des revendications précédentes, dans laquelle l'ester alkylique d'acide gras en C₁-C₄ est alkyle myristate en C₁-C₄.

15. Formulation pharmaceutique selon quelconque des revendications précédentes, dans laquelle l'ester alkylique d'acide gras en C₁-C₄ est isopropyle myristate.

16. Formulation pharmaceutique selon quelconque des revendications précédentes, **caractérisée en ce que** qu'elle est une solution transparente.

17. Kit pour la préparation instantanée d'une formulation pharmaceutique selon quelconque des revendications précédentes, qui comprend un premier récipient qui contient un analogue de la somatostatine comme inhibiteur de la libération de l'hormone de croissance et un deuxième récipient qui contient une solution lipidique qui consiste essentiellement en l'alcool en C₁-C₈, le phospholipide et l'ester alkylique d'acide gras en C₁ -C₄.

18. Kit selon la revendication 17, qui contient de 10 à 60 mg, de préférence de 20 à 40 mg, de l'analogue de la somatostatine comme inhibiteur de la libération de l'hormone de croissance.

19. Kit selon la revendication 17 ou 18, dans lequel l'analogue de la somatostatine comme inhibiteur de la libération de l'hormone de croissance est sous forme lyophilisée.

20. Formulation pharmaceutique selon l'une quelconque des revendications 1-16, pour l'utilisation dans un traitement thérapeutique qui comprend la délivrance prolongée d'un analogue de la somatostatine comme inhibiteur de l'hormone de croissance par une administration unique sous-cutanée ou intramusculaire de la dite composition au moins une fois tous les 20 jours, dé préférence 35 jours.

21. Formulation pharmaceutique selon la revendication 20, dans laquelle l'administration unique sous-cutanée ou intramusculaire de la dite composition est effectuée au moins une fois tous les 40 jours, de préférence 49 jours.

22. Formulation pharmaceutique selon la revendication 20 ou 21, dans laquelle sont administré de 10 à 100 mg/ml de l'analogue de la somatostatine comme inhibiteur de la libération de l'hormone de croissance.

23. Formulation pharmaceutique selon la revendication 22, dans laquelle sont administré de 10 à 60 mg/ml de l'analogue de la somatostatine comme inhibiteur de la libération de l'hormone de croissance.

24. Formulation pharmaceutique selon la revendication 23, dans laquelle est administrée une dose unique de 20 à 40 mg/ml de l'analogue de la somatostatine comme inhibiteur de la libération de l'hormone de croissance.
